# EUROPEAN PATENT APPLICATION

(11) **EP 3 299 050 A1**
(43) Date of publication of application: **28.03.2018**
(21) Application number: 16306229.2
(22) Date of filing: 26.09.2016
(51) Int. Cl.: A61M 5/00, A61M 5/50, B01D 53/04, B65D 75/32, B65D 81/26, A61B 50/30

(54) **PACKAGING FOR A PRE-FILLED SYRINGE, METHOD OF PACKAGING SUCH SYRINGE AND ITS USE**

(71) Applicant: Clariant Healthcare Packaging (France) SAS, 94600 Choisy le Roi (FR)
(72) Inventor: RAULT, Stéphane, 75015 Paris (FR); STOUFFLET, Laurent, 92290 Châtenay-Malabry (FR)
(74) Representative: Hoffmann Eitle

(57) **Abstract**

A tamper-evident pharmaceutical packaging for a pre-filled syringe (20) comprises a rigid housing (12) made of glass or plastic material, the housing (12) being shaped to receive at least one pre-filled syringe (20); the housing forming at least one receiving chamber (52) shaped to receive and physically protect a single pre-filled syringe (20); and having a tip section (34), a barrel section (32) and a plunger section (36), respectively dimensioned to receive the tip (44), the barrel (42) and the plunger (38) of the pre-filled syringe (20); the housing (12) having an access opening (18) to the plunger section of each of the at least one receiving chamber (52); each access opening (18) being arranged to be positioned remote from the tip section (34) of the housing (12); the packaging (10) further comprising at least one openable cover (14), each cover (14) hermetically sealing one of the at least one access openings (18) of the housing (12).

## Description

### Field of the Invention

The invention relates to a tamper evident pharmaceutical packaging for a pre-filled syringe, a method of packaging a pre-filled syringe in such a packaging and the use of such a packaging.

### Prior Art

In the prior art, pre-filled syringes are increasingly used in hospitals because they are quick and fail-safe to use.

Usually, pre-filled syringes are packaged in a blister packaging_{[DH1]}. A conventional blister pack is disclosed in JP 2011-006154 A.

There is a need for a packaging for pre-filled syringes that can insure the preservation of sensitive substances contained in the pre-filled syringe, during an extended shelf life. Furthermore, for multidose syringes, there is a need for a packaging that can insure this preservation while the packaging has to be opened and closed several times. Furthermore, there is a need for a packaging that is tamper-evident.

There is a problem that containers for pre-filled syringes, even tamper-evident blister packaging, are sometimes subject to undetectable theft of the content of the syringe. Especially when syringes contain drugs or narcotic substances, a person having access to the blister packs can use another syringe to withdraw the drug through the packaging and to replace it by another liquid. This is especially problematic because the theft becomes not apparent and the patient is not given the desired dose of the pharmaceutical substance. Therefore, it is desired to design a packaging for a pre-filled syringe that is "secured" such that the integrity of the content of the syringe is protected.

Further, there is a need for a compact packaging in order to meet space constrains in those storage spaces of the hospitals which have to be securely locked in order to avoid inhibited access of any personal to substances the use of which might be subject to legal restrictions.

### Disclosure of the Invention

It is an object of the invention to provide a pharmaceutical packaging for a pre-filled syringe which is both compact and tamper-evident, as well as its use and a method of packaging same.

This object is solved by a tamper-evident packaging for a pre-filled syringe with the features of claim 1, a method of packaging a pre-filled syringe in such a packaging with the features of claim 12 and the use of such packaging according to claim 13. Preferred embodiments follow from the other claims.

A tamper-evident pharmaceutical packaging for a pre-filled syringe comprises a rigid housing made of glass or plastic material, the housing being shaped to receive at least one pre-filled syringe. The housing forms at least one receiving chamber shaped to receive and physically protect a single pre-filled syringe and has a tip section, a barrel section and a plunger section, respectively dimensioned to receive a tip, a barrel and a plunger of a pre-filled syringe. The housing has an access opening to the plunger section of each of the at least one receiving chambers. Each access opening is arranged to be positioned remote from the tip section of the housing. Further, the packaging comprises at least one openable cover, each cover hermetically sealing one of the at least one access openings of the housing.

This packaging is secured because the rigid housing made of glass or plastic material cannot be easily pierced. Further, the receiving chamber is shaped in such a way that specific parts of the receiving chamber are shaped to receive and physically protect specific parts of the syringe. The access opening is arranged at the plunger section of the at least one receiving chamber. In other words, it is remote from the tip section of the receiving chamber so that any unauthorized person cannot easily gain access to the tip of the syringe, because the syringe cannot be turned around within the packaging. Further, the fact that a cover hermetically seals each access opening of the housing allows to protect sensitive substances contained in the pre-filled syringe from the atmosphere external to the housing. Further, the fact that the cover is tamper-evident makes it easily evident in case that the packaging has been opened before. In regular use, the openable cover has to be removed so that any damage to such cover will be noticed. Preferably, the at least one openable cover is non-reclosable. In this manner, the first opening becomes more evident and the packaging is even more secured against undetectable theft.

A further advantage of the pharmaceutical packaging is its strong barrier against the ingress of undesired gaseous components, like oxygen. A rigid housing must have a certain wall thickness to impart the desired strength. The cover hermetically seals each access opening so that oxygen or moisture can also not easily permeate the cover.

The inventive method of packaging a pre-filled syringe in such a packaging comprises the steps of inserting the pre-filled syringe through the access opening into the housing and towards a closed end of the tip section which is arranged opposite to the access opening such that the tip of the pre-filled syringe enters the tip section of the housing, preferably introducing an inert gas or gas mixture, preferably nitrogen, into the access opening of the housing in order to remove remaining air inside the housing, and sealing the access opening by means of the cover such that the cover forms a hermetic seal closing the opening. When introducing an inert gas or gas mixture into the access opening of the housing, the inner atmosphere of the housing can be made oxygen-free before closing the housing by means of the cover. Such step can be desirable in order to extend the shelf life of the packaging.

According to the inventive use, the pre-filled syringe is filled with a liquid or a powder which preferably is a narcotic or pain-killing liquid and most preferably a barbiturate, a benzodiazepine, morphine or adrenalin.

According to a preferred embodiment, the housing forms one receiving chamber for a single pre-filled syringe. Especially in case of the preferred use of the syringe for barbiturates, a single dose is preferred because the administrative requirements like a signature on a list for each pharmaceutical syringe taken from stock can be simplified. In other cases, however, a multi-packaging can be the preferred choice, like single dose syringes against thrombosis for hospital patients who cannot leave their beds. In such a case, a multi-chamber packaging can reduce the required packaging material per syringe and the associated waste, because a plurality of receiving chambers arranged next to each other will at least in part share a common separation wall.

The cover can be made of any material. It preferably comprises plastic and/or aluminum. Preferably, the cover is welded to the housing, preferably at a flat upper surface surrounding the access opening. In this respect, it can be attached to the housing by means of heat welding or heat fusion, ultrasonic welding, induction welding or friction stir welding (FSW).

In general, the cover can be provided by means of a plastic closure that can optionally comprise a hinge connecting the closure to the housing. For example, the hinge can connect directly the closure to the housing or can connect the closure to a retaining ring intended to be assembled on the housing.

According to a preferred embodiment, the cover is a foil hermetically sealed to the housing at a position surrounding the access opening, the foil preferably comprising an aluminum layer. Further, a mono-layer or multi-layer foil can be used. When using a multi-layer foil, it is preferred to combine a layer of aluminum with at least one further layer of plastic material and/or paper material. Examples of a multi-layer foil are aluminium/PE, aluminum/PET, or PET/aluminum/PE.

When such cover is a foil which is hermetically sealed to the housing at a position surrounding the access opening, it is easy to verify whether the receiving chamber has been opened before. Further, the hermetical seal provides air tightness. In order to provide a suitable region surrounding the access opening, the housing can comprise a flange positioned at a position surrounding the access opening.

Further, the foil can be sealed to the housing by means of heat sealing, for example using a seal layer such as Tie, PE or HDPE. Further, it is possible to seal the foil to the housing by means of pressure, for example using a pressure sensitive adhesive.

Preferably, the foil is puncture-proof, which means that it has a certain resistance against puncturing or that makes it visually evident in case that it has been punctured or damaged. In order to increase the resistance against puncture, one side of the foil and preferably the outer side of the foil facing to the outside of the packaging could be provided with a resistant coating. Such resistant coating could be a layer of a rigid material which is supplied on the external side of the foil. Examples of resistant coatings are PET, nylon or a cardboard.

In general, a foil in terms of this description has a thickness not exceeding 1,5mm, preferably 1mm. Preferably, the foil is flexible. For that, a thickness of less than 0,5mm is preferred.

According to a preferred embodiment, the cover has a seizure means not being affixed to the housing. Such seizure means can be an opening tab or a traction ring on the external side of the cover. In such a way, the user can easily grasp the seizure means and open the packaging, even in circumstances when wearing gloves which might be wet.

Preferably, the housing is at least partially transparent. A transparent housing enables a visual inspection at the production site as part of the quality control and after delivery to its destination of use, the packaging can be checked to make sure that it has not been damaged during transport. Suitable plastic materials could be polycarbonates or polyesters.

According to a preferred embodiment of the invention, the housing is made of a plastic composition comprising an active material, preferably a desiccant and/or an oxygen scavenger.

An active material is any substance that is able to trap a gaseous substance, for example oxygen, humidity, odors, carbonates and others. Preferably, the active agent is able to trap oxygen and/or moisture. Examples of sorbents are desiccants or oxygen scavengers. Suitable desiccants are those of the kind of anhydrous salts, reactive desiccants such as calcium oxide, or desiccants which are used for physical adsorption such as molecular sieves, silica gels, clays and starches. Oxygen scavengers of any kind can be used, like iron-based oxygen scavengers, polymer-based oxygens scavengers, enzymatic-based oxygen scavengers, ascorbate, sodium hydrogen carbonate and others.

A suitable material is so-called CESA-absorb which is a registered trademark of Clariant and is a material comprising a polymer and at least an oxygen scavenger.

Such active material can be entrained in the plastic polymer of the housing.

In addition to the use of an active material entrained plastic polymer but also as an alternative, an active material can be positioned inside the housing. The active material is preferably a sorbent and preferably a desiccant and/or an oxygen scavenger. The active material can be housed in a canister or capsule positioned inside the housing or in a compartment that is part of the housing, for example in a chamber positioned in the bottom of the tip section. The active material can also be part of a plastic molded piece that is positioned inside the housing, for example a cylinder molded with an active composition and positioned in the plunger section around the plunger of the syringe. An active material composition can also be applied on the internal side of the cover (for example by coating, laminating, spraying, overmolding...). The provision of an active material inside the housing will, depending on the capacity of the active material, over an extended period of time maintain desired conditions inside the housing, like being dry and/or being nearly free of oxygen. This can considerably increase the shelf time of the product packaged inside the packaging.

According to a preferred embodiment, the housing has a stepped portion which is formed at the end of the barrel section of the housing. Such a stepped portion has the advantage that the gripping plate at the end of the barrel of the syringe can rest against the stepped portion so that the tip of the syringe with or without an attached needle will not abut against the end of the tip section of the housing. Further, such a stepped portion can avoid an insertion of the pre-filled syringe in the wrong direction, i.e. with the plunger first.

In a preferred embodiment of the invention, the housing has at least one flat surface which is arranged to prevent the packaging from inadvertently rolling once the housing is positioned with at least one of the flat surfaces facing a flat supporting surface. When a plurality of packagings are put on a table or sideboard, there is the danger that a packaging could start to roll and drop down. In order to avoid the risk that the pre-filled syringe might become damaged inside the protective packaging, at least one flat surface is provided which either prevents or at least stops an inadvertent rolling of the packaging.

### Brief Description of the Drawings

In the following, preferred embodiments of the invention will be described with reference to the drawings in which
- Fig. 1: describes a longitudinal cut of an inventive protective packaging for a pre-filled syringe;
- Fig. 2a: is an outer view of an inventive packaging; and
- Figs. 2b, 2c: are cross-sectional views in the longitudinal direction of the packaging as shown in Fig. 2a, according to line A-A and line B-B respectively.
- Fig. 2d: is a view from above of the housing as shown in Fig. 2a where the cover has been removed.

### Description of Preferred Embodiments

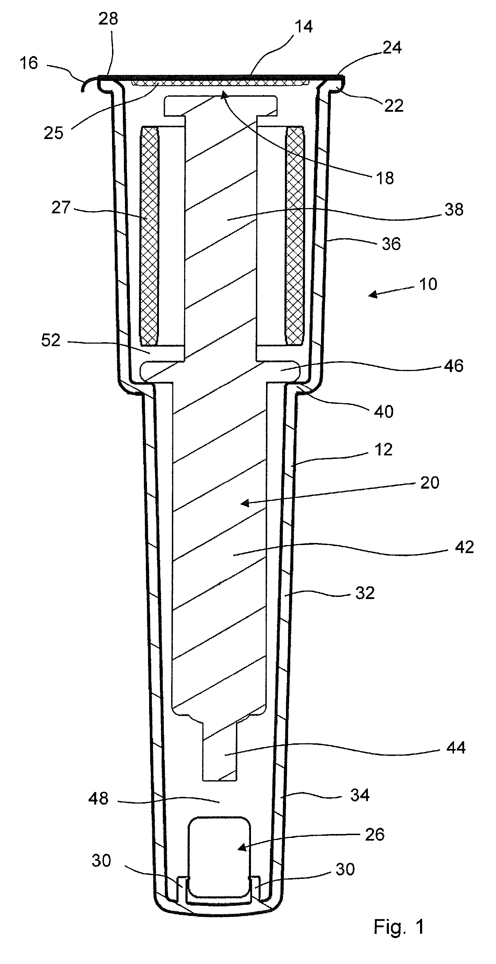

The packaging 10 as shown in Fig. 1 consists of a rigid housing 12 and a cover 14 closing the top side of the housing. In order to conveniently remove the cover 14 from the housing, a flap 16 can be provided which can be easily gripped by the user. Inside the housing, a pre-filled syringe 20 is positioned (in the receiving chamber 52).

The housing consists of a rigid material like glass or plastic material.

Known plastic materials can be used to form the housing 10 and include radical or linear high and low density polyethylenes, copolymers of ethylene such as for example ethylene vinyl acetates, ethylene ethyl acrylates, ethylene butyl acrylates, ethylene maleic anhydrides, ethylene alpha olefines, regard-less of the methods of polymerisation or modification by grafting, homo polypropylene and copolymers, polybutene-1, polyisobutylene. Polyolefines are preferably selected to make the housing 10 for cost reasons and because they are easy to use.
Other polymer materials can be considered however such as polyvinyl chloride, copolymers of vinyl chloride, polyvinylidene chlorides, polystyrenes, copolymers of styrene, derivatives of cellulose, polyamides, polycarbonates, polyoxymethylenes, polyethylene terephthalates, polybutylene terephthalates, copolyesters, polyphenylene oxides, polymethyl methacrylates, copolymers of acrylate, fluoride polymers, polyphenylene sulphides, polyarylsulphones, polyaryletherketones, polyetherimides, polyimides, thermoplastic elastomers, polyurethanes, phenol resins, melamine resins, urea resins, epoxy resins and unsaturated polyester resins.

Biodegradable polymer materials, with for example a starch base, are also possible such as polylactic acids (PLA).

Combinations of these polymers can be used, if desired. The polymer used to produce the housing 10 can also contain one or more additives such as elastomers, fibers, expanding agents, additives such as stabilizers and colorants, sliding agents, demolding agents, adhesion agents or reinforced catching agents and/or any others according to the requirements of usage.

The housing 10 can also be made from injectable blends of materials made in such a way that they are capable of absorbing various different pollutants such as humidity, oxygen, odour and other possible pollutants. The thermoplastic materials are thus themselves formulated with additives belonging to a group of humidity absorbers, oxygen scavengers, odour absorbers and/or emitters of volatile olfactory organic compounds. The formulated thermoplastic materials must however retain a certain degree of resilience.

When it is desired to make the housing of a transparent plastic material, especially polyesters, PET, clarified PP or polycarbonates are preferably used.

On top of the housing 12, an access opening 18 is shown. The access opening is surrounded by a flange 22 which provides a flat upper surface 24 which is used to affix the cover 14 to the flange 22. Inside the housing, an active material can be arranged. It can be housed in a canister or capsule 26 positioned inside the housing 12 or can be part of a plastic moulded piece 27 that is positioned inside the housing. Optionally, an active material composition 25 can also be applied on the internal side of the cover. At those positions, in which an annular seal 28 is formed between the cover 14 and a flange 22 of the housing 12, such active material is preferably not applied. The canister or capsule 26 can be affixed to the housing by means of any suitable fixing structure like claws 30 which can be used to attach the canister 26 inside the housing 12.

The housing 12 is structured into a tip section 34, a barrel section 32 and a plunger section 36. The plunger section 36 houses the plunger 38 of a pre-filled syringe 20, whereas the barrel section 32 houses the barrel 42, and the tip section 44 houses the tip of the syringe 44 which can optionally be provided with a needle or an adaptor or connector for drip.

Between the plunger section 36 and the barrel section 32, a step portion 40 can be provided which has the function to provide an engagement surface for the finger plate 46 of the syringe 20. The step portion 40 is positioned such that there is a predetermined clearance 48 between the tip end of the pre-filled syringe and the housing 12 including a possible canister 26 affixed to that part of the housing. In such a way, the pre-filled syringe rests in the housing such that a needle which can already be attached to the tip of the syringe cannot come into contact with any part of the housing.

The cover 14 is a foil which provides tamper-evidence when the foil has been either taken away, peeled, torn or penetrated in any way before its regular use. Since the user must grip the flap 16 in order to remove the cover 14 from the housing 12, the user will easily recognize any unauthorized tampering with the packaging. When the cover 14 is sealingly attached to the whole circumference of the flange 22 of the housing, it provides a hermetic seal and prevents the ingress of any undesirable substances into the interior of the packaging.

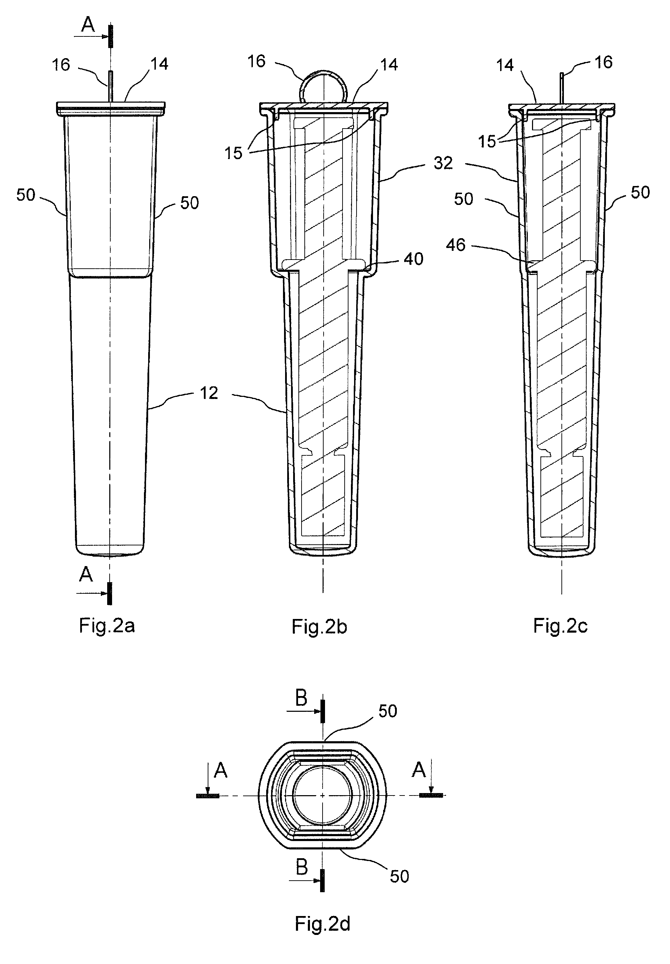

Instead of the foil as shown in Fig. 1, a plastic cover can also be used. In the embodiment as shown in Figs. 2a, 2b, 2c and 2d, a plastic cover sheet 14 is welded to the housing 12 at the flat upper surface 24 surrounding the access opening 18. It can be affixed to the housing by ultrasonic welding, by heat welding, by ultrasound welding or by induction. It can further comprise a sealing skirt 15 that seals against the internal side of the housing and provide a moisture-tight hermetic seal. In this case, the sealing skirt is preferably sufficiently short such that, after the cover has been first removed, the sealing skirt can no more hold the cover on the housing (if the cover is used to reclose the housing). In this manner, the cover is non-reclosable after the housing has been first opened.

In the embodiment as shown in Figs. 2a, 2b, 2c and 2d, a tamper-evident packaging is used which has flattened surfaces 50. Conventional syringes are provided with a type of finger plate 46 which is not provided as an annular ring but has an elliptical shape. As a result of this, the step portion 40 of the housing 12 also does not need to be of an annular shape. It only has to be provided in accordance with the geometry of the finger plate 46 which allows the provision of one or two flattened surfaces 50 at those positions of the barrel section 32 of the housing 12 in which the finger plate of the syringe has a smaller extension. As a result of this, the syringe has to be packaged in a certain annular orientation so that it can be appropriately housed inside the packaging. However, this imposes no problem because the packaging can be carried out in automatic devices and after its single use, the syringes do not have to be put back into the packaging.

The flattened surface or surfaces 50 have the advantage that the packaging cannot inadvertently start to roll and any rolling motion will either be prevented from the very beginning or stop after a slight rolling movement when the packaging will come to rest on one of its flattened surfaces.

In the embodiment according to Figs. 2a to 2d, no canister is included in the packaging. In such a case, the housing can be made of a polymer which has a certain content of an active material which could be a suitable absorbent material.

The inventive tamper-evident pharmaceutical packaging has the advantages that it is compact, not easily perforated in the region of the tip of the syringe and not accessible from the access opening without removing the syringe from the packaging which implies an opening of the cover 14 which can be easily detected.

## Claims

1. Tamper-evident pharmaceutical packaging for a pre-filled syringe (20), comprising:
- a rigid housing (12) made of glass or plastic material,
- the housing (12) being shaped to receive at least one pre-filled syringe (20);
- the housing forming at least one receiving chamber (52) shaped to receive and physically protect a single pre-filled syringe (20); and having a tip section (34), a barrel section (32) and a plunger section (36), respectively dimensioned to receive the tip (44), the barrel (42) and the plunger (38) of the pre-filled syringe (20);
- the housing (12) having an access opening (18) to the plunger section of each of the at least one receiving chamber (52);
- each access opening (18) being arranged to be positioned remote from the tip section (34) of the housing (12);
- the packaging (10) further comprising at least one openable cover (14), each cover (14) hermetically sealing one of the at least one access openings (18) of the housing (12).

2. Packaging according to claim 1, wherein the housing (12) forms one receiving chamber (52) for a single pre-filled syringe (20).

3. Packaging according to claim 1 or 2,
**characterized in that**
the cover (14) is a foil hermetically sealed to the housing (12) at a position (22) surrounding the access opening (18), the foil preferably comprising an aluminum layer.

4. Packaging according to any of the preceding claims,
**characterized in that**
the cover (14) is welded to the housing (12) at a flat upper surface (24) surrounding the access opening (18).

5. Packaging according to any of the preceding claims,
**characterized in that**
the corresponding access opening (18) is not reclosable by the cover (14) once the cover (14) is removed.

6. Packaging according to any of the preceding claims, the cover (14) having seizure means (16) not being affixed to the housing (12).

7. Packaging according to any of the preceding claims,
**characterized in that**
the housing (12) is at least partially transparent.

8. Packaging according to any of the preceding claims,
**characterized in that**
the housing (12) is made of a plastic composition comprising an active material, preferably a desiccant and/or an oxygen scavenger.

9. Packaging according to any of the preceding claims, further comprising an active material positioned inside the housing, the active material preferably being a sorbent and most preferably an oxygen scavenger.

10. Packaging according to claim 8 or 9, wherein the oxygen scavenger is an iron-based material or a polymer-based material, preferably a polymer-based oxygen scavenger.

11. Packaging according to any of the preceding claims, the housing (12) having a stepped portion (40) which is formed at the end of the barrel section (32) of the housing (12).

12. Packaging according to any of the preceding claims, the housing (12) having at least one flat surface (50) which is arranged to prevent the packaging (10) from inadvertently rolling once the housing (12) is positioned with at least one of the flat surfaces facing a flat supporting surface (50).

13. Method of packaging a pre-filled syringe in a packaging according to any of the preceding claims, comprising the steps of:
- inserting the pre-filled syringe (20) through the access opening (18) into the housing (12) and towards a closed end of the tip section (34) which is arranged opposite to the access opening (18) such that the tip of the tip (44) of the pre-filled syringe (20) enters the tip section (34) of the housing (12);
- preferably introducing an inert gas or gas mixture, preferably nitrogen, into the access opening (18) of the housing (12) in order to remove remaining air inside the housing (12); and
- sealing the access opening (18) by means of the cover (16) such that the cover (16) forms a hermetic seal closing the access opening (18).

14. Use of a packaging according to any of the claims 1 to 11 for storing a pre-filled syringe (20) filled with a liquid or a powder, preferably narcotic or pain-killing liquid, most preferably a barbiturate.
